# DEMANDE DE BREVET EUROPEEN

(11) **EP 3 320 809 A1**
(43) Date de publication de la demande: **16.05.2018**
(21) Numéro de dépôt: 17200160.4
(22) Date de dépôt: 06.11.2017
(51) Int. Cl.: A47C 31/10, A47C 21/00, A47C 31/00, A47D 15/00, G08B 21/02, A61B 5/00, A61B 5/113

(54) **DISPOSITIF DE COUCHAGE ET INSTALLATION DE COUCHAGE COMPRENANT UN TEL DISPOSITIF DE COUCHAGE**

(30) Priorité: 14.11.2016 FR 1660957
(71) Demandeur: Plasti Temple, 49015 Angers (FR)
(72) Inventeur: JAMET, Frédéric, 49800 Trelaze (FR)
(74) Mandataire: Godineau, Valérie

(57) **Abrégé**

Dispositif (1) de couchage comprenant un matelas (2) et un boîtier (12) électronique, ledit matelas (2) comprenant une âme (6) et une housse (7) munie d'une ouverture (8) d'insertion de l'âme (6) dans la housse (7) ;

La housse (7) comprend intérieurement une poche (10) couplée, à la manière d'un volet, à la housse (7) par une liaison (11) charnière d'axe pivot sensiblement parallèle à la ligne de fermeture formée par la portion de la fermeture (9) à glissière s'étendant le long d'un flanc (5) longitudinal du matelas (2), ladite poche (10) logeant de manière amovible le boîtier (12) électronique, ledit boîtier (12) électronique comprenant un corps (13) délimitant une enceinte (14) apte à communiquer avec l'extérieur.

## Description

### DOMAINE DE L'INVENTION

L'invention concerne un dispositif de couchage et une installation de couchage comprenant un tel dispositif de couchage.

Elle concerne plus particulièrement un dispositif de couchage comprenant un matelas et un boîtier électronique, ledit matelas comprenant une âme et une housse munie d'une ouverture d'insertion de l'âme dans la housse, cette ouverture de la housse étant obturable par une fermeture à glissière, ledit matelas présentant, à l'état inséré de l'âme dans la housse, une première face et une deuxième face aptes à former indifféremment l'une, la face de dessus, l'autre, la face de dessous du matelas et des flancs de liaison des première et deuxième faces entre elles, la fermeture à glissière s'étendant au moins longitudinalement sur un flanc longitudinal du matelas à écartement des première et deuxième faces du matelas.

### ART ANTERIEUR

Un tel dispositif de couchage destiné en particulier aux nourrissons et jeunes enfants est connu comme l'illustre par exemple le brevet US 2012/053424. Le document US 2016/206240 décrit également une housse de matelas logeant de manière amovible un boîtier électronique. Il en est de même du document US 2008/106421. De nombreux parents souhaitent qu'un tel dispositif de couchage permette une surveillance discrète de l'enfant sans perdre ses qualités de confort en termes de couchage et la possibilité d'utiliser un tel dispositif de couchage avec l'une de ses faces servant de surface de couchage sur une première période d'âge de l'enfant et l'autre face servant de surface de couchage sur une deuxième période d'âge de l'enfant.

### BUTS ET RESUME

Un but de l'invention est donc de proposer un dispositif de couchage dont la conception permet, en dépit de la réversibilité du matelas, l'insertion de manière sûre et discrète d'un boîtier électronique.

A cet effet, l'invention a pour objet un dispositif de couchage comprenant un matelas et un boîtier électronique, ledit matelas comprenant une âme et une housse munie d'une ouverture d'insertion de l'âme dans la housse, cette ouverture de la housse étant obturable par une fermeture à glissière, ledit matelas présentant, à l'état inséré de l'âme dans la housse, une première face et une deuxième face aptes à former indifféremment l'une, la face de dessus, l'autre, la face de dessous du matelas et des flancs de liaison des première et deuxième faces entre elles, la fermeture à glissière de la housse s'étendant au moins longitudinalement sur un flanc longitudinal du matelas à écartement des première et deuxième faces du matelas, caractérisé en ce que la housse comprend intérieurement une poche couplée à la manière d'un volet à la housse par une liaison charnière d'axe pivot sensiblement parallèle à la ligne de fermeture formée par la portion de la fermeture à glissière s'étendant le long d'un flanc longitudinal du matelas, pour permettre à la poche d'occuper sélectivement au moins une première position dans laquelle elle s'étend entre la première face du matelas et l'âme, et une deuxième position dans laquelle elle s'étend entre la deuxième face du matelas et l'âme, ladite poche logeant de manière amovible le boîtier électronique, ledit boîtier électronique comprenant un corps délimitant une enceinte, cette enceinte, apte à communiquer avec l'extérieur par l'intermédiaire d'orifices traversants ménagés dans le corps du boîtier, renfermant au moins un module de communication par liaison sans fil apte à communiquer avec au moins un terminal déporté et au moins un capteur de mesure et/ou de détermination d'un paramètre représentatif de l'air environnant et/ou un capteur de mouvement.

La présence d'une poche positionnable indifféremment le long de l'une ou l'autre des faces aptes à former une surface de couchage du matelas permet un positionnement du boîtier électronique au plus près de la personne couchée quelle que soit la face de couchage sélectionnée. Cette poche permet en outre, un positionnement précis du boîtier électronique garantissant un positionnement fiable du ou des capteurs et un logement amovible du boîtier autorisant ainsi de manière aisée son rechargement hors du matelas.

Selon un mode de réalisation de l'invention, la housse est perméable à l'air. Cette perméabilité à l'air permet un fonctionnement optimal de capteurs d'analyse de l'air ambiant.

Selon un mode de réalisation de l'invention, la housse du matelas est élastiquement déformable au moins dans le sens de l'épaisseur. Cette réalisation de la housse de manière élastiquement déformable permet une meilleure transmission des vibrations, résultant de mouvements de la personne couchée sur la surface de couchage, jusqu'au boîtier électronique.

Selon un mode de réalisation de l'invention, dans lequel le dispositif de couchage comprend un capteur de mouvement, ledit capteur de mouvement est un accéléromètre. Cet accéléromètre permet de détecter les mouvements de la personne couchée sur la surface de couchage.

Selon un mode de réalisation de l'invention dans lequel le dispositif de couchage comprend au moins un capteur de mesure et/ou de détermination d'un paramètre représentatif de l'air environnant, le ou au moins l'un des capteurs est choisi dans le groupe formé par les capteurs de mesure de température, les capteurs de mesure d'humidité, les capteurs de détection de la présence d'un gaz.

Selon un mode de réalisation de l'invention, le boîtier renferme en outre au moins un capteur de sons. Un tel capteur de sons permet de déterminer le niveau sonore dans la zone de couchage, une variation du niveau sonore pouvant résulter d'un comportement bruyant de la personne couchée ou d'une variation de l'environnement dans la zone de couchage.

Selon un mode de réalisation de l'invention, le boîtier renferme en outre une unité de pilotage et un accumulateur d'énergie apte à alimenter en énergie ladite unité de pilotage, cette unité de pilotage étant configurée pour acquérir les signaux du ou des capteurs, comparer, si nécessaire, les signaux du ou des capteurs avec des valeurs seuil, et émettre des signaux de sortie aptes à être transmis par l'intermédiaire du module de communication par liaison sans fil à au moins un terminal déporté. La visualisation de telles informations sur le terminal déporté permet de contrôler à distance, de manière simple et non intrusive, ce qui se passe dans la zone de couchage.

Selon un mode de réalisation de l'invention, la poche est couplée à la housse au niveau de la fermeture à glissière.

Dans ce mode de réalisation de l'invention, de préférence, la poche et la fermeture à glissière sont respectivement couplées à la housse par couture, la couture de liaison de la poche à la housse étant de préférence commune à l'une des coutures de liaison de la fermeture à glissière à la housse. Il en résulte une simplicité de fabrication.

Selon un mode de réalisation de l'invention, la poche est formée de deux faces au moins partiellement en regard l'une de l'autre et reliées entre elles sur trois de leurs bords, l'une des faces se prolongeant pour s'étendre au-delà du bord libre de la face en regard et former la partie de liaison de la poche à la housse

Selon un mode de réalisation de l'invention, l'âme du matelas est formée d'une plaque perforée en matière élastiquement déformable et d'un bloc de mousse positionnables en applique l'un contre l'autre, et la poche est configurée pour pouvoir occuper une troisième position dans laquelle elle s'étend entre la plaque et le bloc de mousse de l'âme. Une telle configuration de l'âme permet une adaptation de la surface de couchage en fonction de l'âge de l'enfant, l'utilisation de la plaque comme surface de couchage étant plus particulièrement réservée aux enfants plus âgés. La possibilité d'insérer la poche et donc le boîtier entre la plaque et le bloc permet de rendre moins visible et moins inconfortable la présence du boîtier dans la configuration où la plaque est utilisée comme surface de couchage.

L'invention a encore pour objet une installation de couchage comprenant un dispositif de couchage, un terminal déporté et au moins une application informatique exécutable dans ledit terminal déporté, caractérisé en ce que le dispositif de couchage est du type précité.

### BREVE DESCRIPTION DES DESSINS

L'invention sera bien comprise à la lecture de la description suivante d'exemples de réalisation, en référence aux dessins annexés dans lesquels :

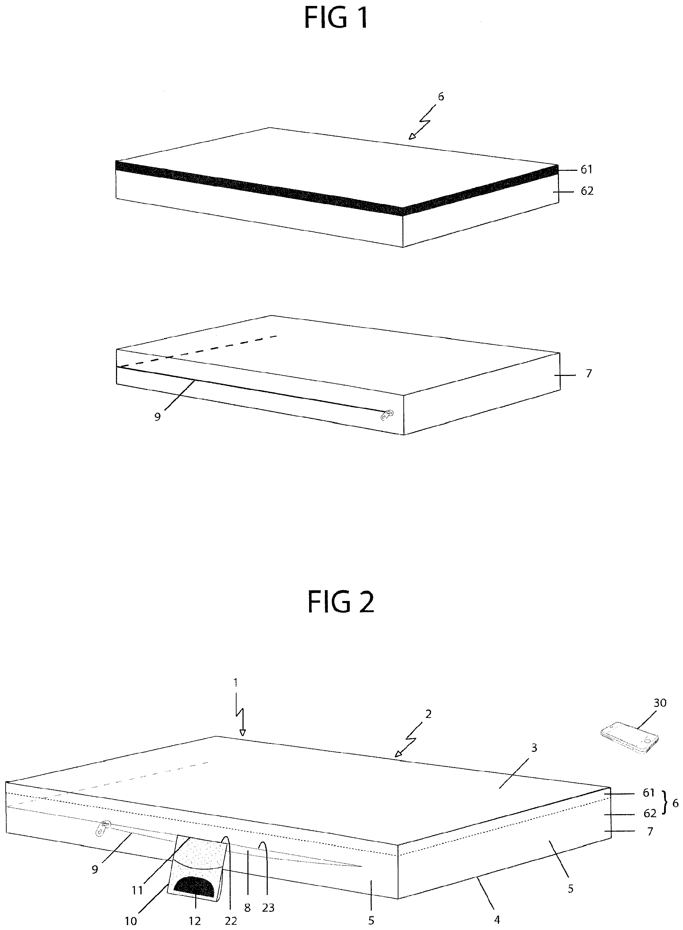

La figure 1 représente une vue de la housse et de l'âme à l'état séparé l'une de l'autre
La figure 2 représente une vue schématique en perspective d'un dispositif de couchage conforme à l'invention en position sortie de la poche
La figure 3 représente une série de vues schématiques en perspective d'un dispositif de couchage conforme à l'invention illustrant les différentes possibilités de positionnement de la poche
La figure 4 représente une vue en perspective du boîtier électronique
La figure 5 représente une vue en perspective en position éclatée des éléments le constituant du boîtier de la figure 4.

### DESCRIPTION DETAILLEE

Comme mentionné ci-dessus, l'invention a pour objet une dispositif 1 de couchage et une installation de couchage comprenant un tel dispositif 1 de couchage. Le dispositif de couchage est plus particulièrement destiné aux nourrissons et aux enfants

Le dispositif 1 de couchage comprend un matelas 2 et un boîtier 12 électronique. Le matelas 2 comprend une âme 6 et une housse 7 à l'intérieur de laquelle l'âme 6 est insérable. A l'état inséré de l'âme 6 dans la housse 7, le matelas 2 présente une première face 3 et une deuxième face 4 aptes à former indifféremment l'une, la face de dessus, l'autre, la face de dessous du matelas et des flancs 5 de liaison des première et deuxième faces entre elles. La face de dessus du matelas 2 forme la surface de couchage du matelas. Comme le matelas 2 est un matelas réversible, les première et deuxième faces du matelas peuvent former tour à tour la face du dessus et donc la surface de couchage du matelas. Cette réversibilité permet au matelas de s'adapter à la croissance de l'enfant. En effet, dans l'exemple représenté, l'âme 6 du matelas 2 comprend une plaque 61 micro-perforée en matière élastiquement déformable, en particulier en latex, et un bloc 62 de mousse de polyuréthane de forme générale parallélépipédique. Le bloc 62 de mousse est d'épaisseur supérieure à celle de la plaque 61 et la masse volumique du bloc de mousse est inférieure à celle de la plaque 61. La plaque 61 s'étend en regard de la partie de la housse formant la première face 3 du matelas tandis que le bloc 62 de mousse s'étend en regard de la housse formant la deuxième face 4 du matelas. Ainsi, la plaque 61 est destinée à former la surface de couchage, donc à être positionnée au-dessus du bloc, lorsque le dispositif de couchage est utilisé pour de jeunes enfants. A l'inverse, le bloc 62 est destiné à former la surface de couchage, donc à être positionné au-dessus de la plaque 61, lorsque le dispositif de couchage est utilisé pour les bébés. Le simple retournement du matelas permet de passer d'une configuration à une autre.

La housse 7 d'enveloppement de l'âme 6 est, quant à elle, une housse perméable à l'air, de préférence en latex ou en polyester. Cette housse est de préférence réalisée en un matériau lavable en machine. Dans le cas d'une housse en latex, cette housse est réalisée élastiquement déformable au moins dans le sens de l'épaisseur. Dans le cas d'une housse 7 en polyester, cette housse est une housse réalisée en un tissu à mailles dites tridimensionnelles pour former un tissu alvéolaire. A nouveau, cette réalisation rend la housse 7 élastiquement déformable au moins dans le sens de l'épaisseur.

La housse 7 est munie d'une ouverture 8 d'insertion de l'âme 6 dans la housse 7. Cette ouverture 8 de la housse 7 est obturable par une fermeture 9 à glissière. Cette fermeture 9 à glissière s'étend ici longitudinalement sur un flanc 5 longitudinal du matelas et sur un flanc transversal du matelas pour permettre une mise en place et un enlèvement aisés de l'âme 6. Cette fermeture 9 à glissière est disposée à écartement, ici à mi-distance des première et deuxième faces du matelas à l'état inséré de l'âme 6 dans la housse 7.

La housse 7 comprend encore intérieurement une poche 10 couplée, à la manière d'un volet, à la housse 7 par une liaison 11 charnière d'axe pivot sensiblement parallèle à la ligne de fermeture formée par la portion de la fermeture 9 à glissière s'étendant le long d'un flanc 5 longitudinal du matelas. Cette poche 10 est formée de deux faces au moins partiellement en regard l'une de l'autre et reliées entre elles sur trois de leurs bords, pour former la partie active de la poche. L'une des faces de la poche 10 se prolonge pour s'étendre au-delà du bord libre de la face en regard et former la partie de liaison de la poche à la housse. Dans l'exemple représenté aux figures, la poche 10 est couplée à la housse au niveau de la fermeture 9 à glissière. En particulier, la poche 10 et la fermeture 9 à glissière sont respectivement couplées à la housse par couture. La couture 22 de liaison de la poche 10 à la housse 7 est formée par l'une des coutures 23 de liaison de la fermeture 9 à glissière à la housse 7. Ainsi, en une seule opération de couture, la poche et la fermeture à glissière peuvent être solidarisées à la housse. La ligne de couture reliant la poche 10 à la housse 7 forme la liaison pivot de raccordement de la poche à la housse. Cette ligne de couture s'étend donc au niveau du prolongement de l'une des faces de la poche.

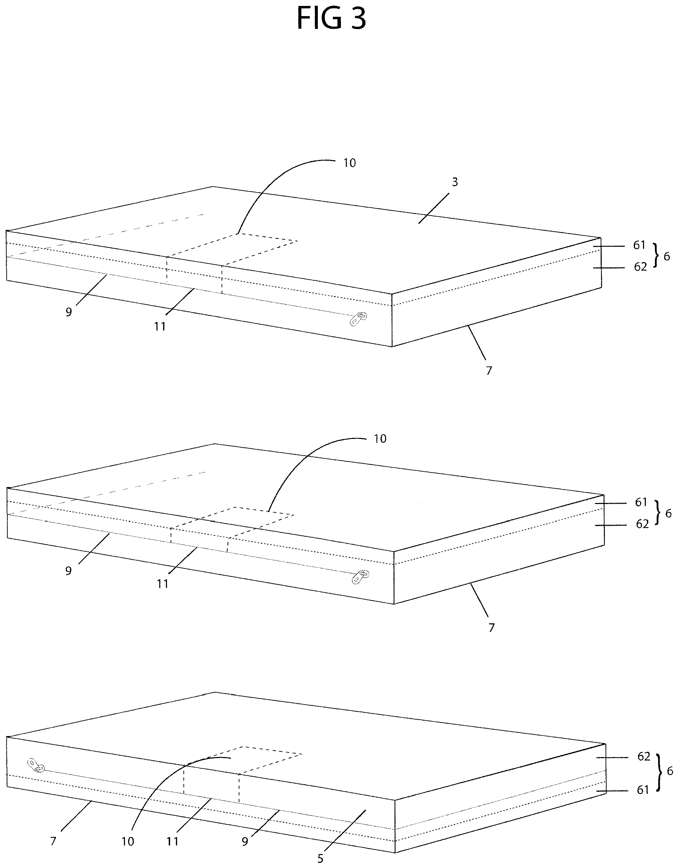

Grâce à cette liaison pivot obtenue ici par couture entre poche 10 et housse 7, et la réalisation en un matériau souple de la poche 10, la poche 10 peut occuper sélectivement plusieurs positions comme illustré à la figure 3. Ainsi, dans l'exemple de la figure 3, la première vue illustre une première position de la poche dans laquelle elle s'étend entre la première face 3 du matelas 2 et l'âme 6, en particulier la plaque 61 de l'âme, cette première face 3 du matelas 2 formant ici en coopération avec la plaque 61, la surface de couchage du matelas. Cette configuration est utilisée pour des enfants dont le poids est supérieur à 10 kg. La dernière vue de la figure 3 illustre une deuxième position de la poche dans laquelle elle s'étend, entre la deuxième face 4 du matelas et l'âme 6, en particulier le bloc 62 de l'âme, la deuxième face 4 du matelas 2 formant ici, en coopération avec le bloc 62 de l'âme, la surface de couchage du matelas. Cette configuration est utilisée pour des bébés dont le poids est inférieur à 10 kg. Enfin, la vue du milieu de la figure 3 illustre une troisième position de la poche dans laquelle elle s'étend entre la plaque 61 et le bloc 62 de l'âme 6, la première face 3 du matelas 2 formant ici en coopération avec la plaque 61, la surface de couchage du matelas. Cette configuration peut également être utilisée pour des enfants dont le poids est supérieur à 10 kg et permet, par comparaison avec la première vue de la figure 3, de rendre moins accessible le contenu de la poche 10.

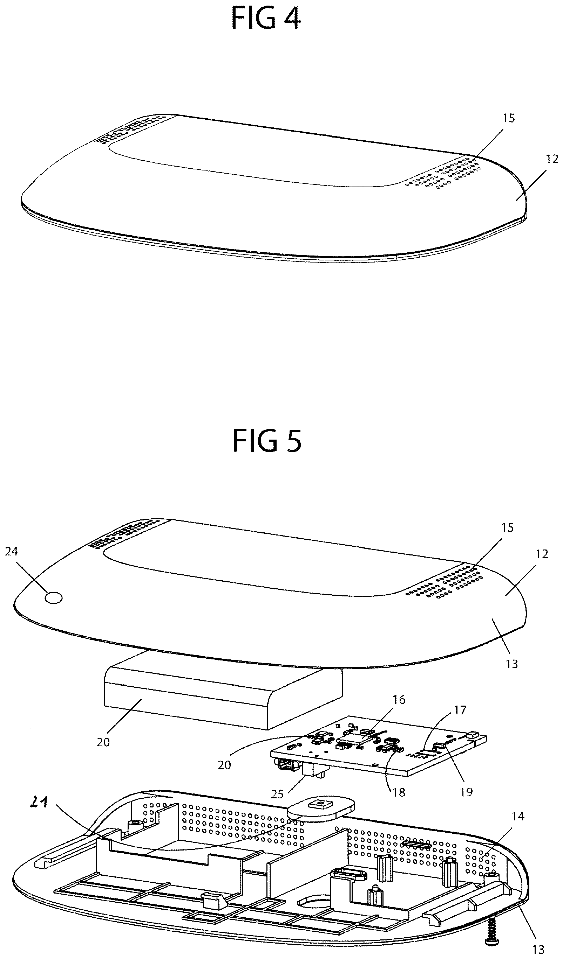

Cette poche 10 est destinée à loger, de manière amovible, un boîtier 12 électronique, tel qu'illustré aux figures 4 et 5. Ce boîtier 12 électronique comprend un corps 13 ici en deux éléments assemblables par un plan de joint Ces éléments délimitent, à l'état assemblé, une enceinte 14. Cette enceinte 14 est apte à communiquer avec l'extérieur par l'intermédiaire d'orifices 15 traversants ménagés dans le corps 13 du boîtier 12. Ce boîtier 12 renferme un module 16 de communication par liaison sans fil apte à communiquer avec au moins un terminal 30 déporté et un ou plusieurs capteurs. Dans l'exemple représenté, le module 16 de communication par liaison sans fil est un module permettant une liaison radio par exemple de type bluetooth ou WIFI avec le terminal 30 déporté. Ce terminal 30 déporté peut être un téléphone portable ou une tablette informatique, un ordinateur personnel ou autre.

Ce boîtier 12, qui se glisse simplement dans la poche 10, renferme encore plusieurs capteurs 17 de mesure et/ou de détermination d'un paramètre représentatif de l'air environnant. Ainsi, dans l'exemple représenté, le boîtier 12 renferme un capteur de mesure de la température de l'air, un capteur de mesure de l'humidité de l'air et un capteur de mesure de la qualité de l'air et en particulier de détection de la présence de monoxyde de carbone. Ce boîtier renferme également un capteur 19 de sons, tel qu'un microphone, et un capteur 18 de mouvement, tel qu'un accéléromètre.

Pour parfaire l'ensemble, le boîtier 12 renferme en outre une unité 20 de pilotage et un accumulateur 21 d'énergie, en l'occurrence une batterie, apte à alimenter en énergie ladite unité 20 de pilotage. Cette unité 20 de pilotage comprend des moyens de traitement électroniques et/ou informatiques des données, tels qu'un microprocesseur associé à une mémoire de travail. Cette unité 20 de pilotage est configurée pour acquérir les signaux du ou des capteurs, comparer les signaux desdits capteurs avec des valeurs seuil et émettre des signaux de sortie aptes à être transmis par l'intermédiaire du module 16 de communication par liaison sans fil à au moins un terminal 30 déporté, tel que le téléphone des parents. Ce terminal 30 déporté inclut une application informatique exécutable par le terminal. Cette application informatique comprend un ensemble d'instructions informatiques qui permettent d'exécuter des fonctions de requête d'informations, d'affichage des informations fournies par lesdits capteurs, d'émission de signaux d'alerte visuels ou sonores.

Ainsi, grâce à la présence des différents capteurs, il est possible d'afficher sur l'écran d'affichage du terminal déporté, la température, le taux d'humidité, la qualité de l'air de la zone de couchage, un suivi en temps réel du sommeil de la personne couchée, le niveau sonore de la zone de couchage, le niveau de la batterie et d'émettre si nécessaire des signaux d'alerte. Il en résulte la possibilité de surveiller à distance la zone de couchage.

Dans l'exemple représenté, le boîtier 12 est équipé d'un bouton 24 marche/arrêt et d'une connectique électrique 25 pour le raccordement du boîtier 12 à une source d'énergie extérieure en vue du rechargement de la batterie. Les parents peuvent donc à tout moment arrêter la surveillance par actionnement du bouton marche/ arrêt. Le rechargement nécessite quant à lui la sortie du boîtier de la poche.

Le fonctionnement d'un tel dispositif de couchage pour enfants ou nourrissons est tel que suit : les responsables de l'enfant, par exemple ses parents, décident de la surface de couchage du matelas à utiliser en fonction de l'âge de l'enfant. On suppose que l'enfant est un nourrisson et que la surface de couchage est formée par le bloc 62 de l'âme comme illustré à la dernière vue de la figure 3. Le boîtier 12 est allumé par actionnement du bouton marche/arrêt et glissé dans la poche. Cette poche est positionnée entre le bloc et la housse au niveau de la première face 3 du matelas. Les données fournies par les capteurs peuvent alors être émises directement ou après traitement en direction d'un terminal déporté formé par exemple par le téléphone des parents. Les parents sont ainsi constamment informés de ce qui se passe dans la zone de couchage.

## Revendications

1. Dispositif (1) de couchage comprenant un matelas (2) et un boîtier (12) électronique, ledit matelas (2) comprenant une âme (6) et une housse (7) munie d'une ouverture (8) d'insertion de l'âme (6) dans la housse (7), cette ouverture (8) de la housse (7) étant obturable par une fermeture (9) à glissière, ledit matelas (2) présentant, à l'état inséré de l'âme (6) dans la housse (7), une première face (3) et une deuxième face (4) aptes à former indifféremment l'une, la face de dessus, l'autre, la face de dessous du matelas (2) et des flancs (5) de liaison des première et deuxième faces (3, 4) entre elles, la fermeture (9) à glissière de la housse (7) s'étendant au moins longitudinalement sur un flanc (5) longitudinal du matelas (2) à écartement des première et deuxième faces du matelas (2),
**caractérisé en ce que** la housse (7) comprend intérieurement une poche (10) couplée, à la manière d'un volet, à la housse (7) par une liaison (11) charnière d'axe pivot sensiblement parallèle à la ligne de fermeture formée par la portion de la fermeture (9) à glissière s'étendant le long d'un flanc (5) longitudinal du matelas (2) pour permettre à la poche (10) d'occuper sélectivement, au moins une première position dans laquelle elle s'étend entre la première face (3) du matelas (2) et l'âme (6), et une deuxième position dans laquelle elle s'étend entre la deuxième face (4) du matelas (2) et l'âme (6), ladite poche (10) logeant de manière amovible le boîtier (12) électronique, ledit boîtier (12) électronique comprenant un corps (13) délimitant une enceinte (14), cette enceinte (14), apte à communiquer avec l'extérieur par l'intermédiaire d'orifices (15) traversants ménagés dans le corps (13) du boîtier (12), renfermant au moins un module (16) de communication par liaison sans fil apte à communiquer avec au moins un terminal déporté et au moins un capteur (17) de mesure et/ou de détermination d'un paramètre représentatif de l'air environnant et/ou un capteur (18) de mouvement.

2. Dispositif (1) de couchage selon la revendication 1,
**caractérisé en ce que** la housse (7) est perméable à l'air.

3. Dispositif (1) de couchage selon l'une des revendications précédentes,
**caractérisé en ce que** la housse (7) est élastiquement déformable au moins dans le sens de l'épaisseur.

4. Dispositif (1) de couchage selon l'une des revendications précédentes du type comprenant un capteur (18) de mouvement, **caractérisé en ce que** le capteur (18) de mouvement est un accéléromètre.

5. Dispositif (1) de couchage selon l'une des revendications précédentes du type comprenant au moins un capteur (17) de mesure et/ou de détermination d'un paramètre représentatif de l'air environnant, **caractérisé en ce que** le ou au moins l'un des capteurs (17) est choisi dans le groupe formé par les capteurs de mesure de température, les capteurs de mesure d'humidité et les capteurs de détection de la présence d'un gaz.

6. Dispositif (1) de couchage selon l'une des revendications précédentes,
**caractérisé en ce que** le boîtier (2) renferme en outre au moins un capteur (19) de sons.

7. Dispositif (1) de couchage selon l'une des revendications précédentes,
**caractérisé en ce que** le boîtier (2) renferme en outre une unité (20) de pilotage et un accumulateur (21) d'énergie apte à alimenter en énergie ladite unité (20) de pilotage, cette unité (20) de pilotage étant configurée pour acquérir les signaux du ou des capteurs (17, 18, 19), comparer, si nécessaire, les signaux du ou des capteurs(17, 18, 19) avec des valeurs seuil et émettre des signaux de sortie aptes à être transmis par l'intermédiaire du module (16) de communication par liaison sans fil à au moins un terminal (30) déporté.

8. Dispositif (1) de couchage selon l'une des revendications précédentes,
**caractérisé en ce que** la poche (10) est couplée à la housse (7) au niveau de la fermeture (9) à glissière.

9. Dispositif (1) de couchage selon la revendication précédente, **caractérisé en ce que** la poche (10) et la fermeture (9) à glissière sont respectivement couplées à la housse (7) par couture, la couture (22) de liaison de la poche (10) à la housse (7) étant, de préférence, commune à l'une des coutures (23) de liaison de la fermeture (9) à glissière à la housse (7).

10. Dispositif (1) de couchage selon l'une des revendications précédentes,
**caractérisé en ce que** la poche (10) est formée de deux faces au moins partiellement en regard l'une de l'autre et reliées entre elles sur trois de leurs bords, l'une des faces se prolongeant pour s'étendre au-delà du bord libre de la face en regard et former la partie de liaison de la poche (10) à la housse (7).

11. Dispositif (1) de couchage selon l'une des revendications précédentes,
**caractérisé en ce que** l'âme (6) du matelas (2) est formée d'une plaque (61) perforée en matière élastiquement déformable et d'un bloc (62) de mousse positionnables en applique l'un contre l'autre, et **en ce que** la poche (10) est configurée pour pouvoir occuper une troisième position dans laquelle elle s'étend entre la plaque (61) et le bloc (62) de mousse de l'âme (6).

12. Installation de couchage comprenant un dispositif (1) de couchage, un terminal déporté et au moins une application informatique exécutable dans ledit terminal déporté, **caractérisé en ce que** le dispositif (1) de couchage est conforme à l'une des revendications 1 à 11.
